(19)

**Europäisches Patentamt**
**European Patent Office**
**Office européen des brevets**

(11)  **EP 4 328 242 A1**

(12)  **EUROPEAN PATENT APPLICATION**
published in accordance with Art. 153(4) EPC

(43) Date of publication:
**28.02.2024  Bulletin 2024/09**

(21) Application number: **22791882.8**

(22) Date of filing: **10.03.2022**

(51) International Patent Classification (IPC):
*C07K 16/22* (2006.01)    *A61P 35/00* (2006.01)
*A61P 25/28* (2006.01)    *A61P 27/02* (2006.01)
*C07K 16/28* (2006.01)    *A61K 39/00* (2006.01)

(52) Cooperative Patent Classification (CPC):
**A61K 39/00; A61P 25/28; A61P 27/02;**
**A61P 35/00; C07K 16/22; C07K 16/28**

(86) International application number:
**PCT/KR2022/003351**

(87) International publication number:
**WO 2022/225182 (27.10.2022 Gazette 2022/43)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **23.04.2021  KR 20210052895**
**07.03.2022  KR 20220028751**

(71) Applicant: **Neortesbio Inc.**
**Seongnam-si, Gyeonggi-do 13488 (KR)**

(72) Inventor: **KIM, Yong In**
**Seongnam-si Gyeonggi-do 13556 (KR)**

(74) Representative: **FRKelly**
**Waterways House**
**Grand Canal Quay**
**Dublin D02 PD39 (IE)**

Remarks:
The complete document including Reference
Table(s) and the Sequence Listing(s) can be
downloaded from the EPO website

(54)  **ANTIBODY SPECIFICALLY BINDING TO ANGIOPOIETIN-2, OR FRAGMENT THEREOF**

(57)    The present invention relates to an anti-angiopoietin-2 (Ang2) antibody or an antigen-binding fragment thereof that specifically binds to Ang2 and induces Tie2 activation, comprising: (a) a heavy-chain complementarity-determining region (CDR) comprising CDRH1 of the amino acid sequence of SEQ ID NO: 2, SEQ ID NO: 10, SEQ ID NO: 18, or SEQ ID NO: 26, CDRH2 of the amino acid sequence of SEQ ID NO: 3, SEQ ID NO: 11, SEQ ID NO: 19, or SEQ ID NO: 27, and CDRH3 of the amino acid sequence of SEQ ID NO: 4, SEQ ID NO: 12, SEQ ID NO: 20, or SEQ ID NO: 28; and (b) a light-chain CDR comprising CDRL1 of the amino acid sequence of SEQ ID NO: 5, SEQ ID NO: 13, SEQ ID NO: 21, or SEQ ID NO: 29, CDRL2 of the amino acid sequence of SEQ ID NO: 6, SEQ ID NO: 14, SEQ ID NO: 22, or SEQ ID NO: 30, and CDRL3 of the amino acid sequence of SEQ ID NO: 7, SEQ ID NO: 15, SEQ ID NO: 23, or SEQ ID NO: 31.

【Fig. 3】

EP 4 328 242 A1

**Description**

[Technical Field]

The present invention relates to an anti-angiopoietin-2 (Ang2) antibody or fragment thereof that specifically binds to angiopoietin-2 (Ang2), an angiogenesis-inducing factor, and binds to the Tie2 receptor together with Ang2.

[Background Art]

The angiopoietin protein group is a group of proteins that play an important role in the angiogenesis and maintenance of blood vessels, and there are four types of angiopoietins (Ang1, Ang2, Ang3, and Ang4).

Angiopoietin-1 binds to the Tie2 receptor and plays an important role in vascular maturation, adhesion, migration, and survival.

On the other hand, angiopoietin-2 binds to the receptor Tie2 present in vascular endothelial cells, but by acting as an antagonistic ligand, it acts to inhibit signal transduction by Tie2 by competing for Tie2 binding with Angiopoietin-1 (Ang1), an agonist of Tie2. Due to this mechanism of action, under overexpression of VEGF or inflammation, vascular endothelial cells are activated and vascular permeability increases. In this case, Ang1 induces stabilization of vascular endothelial cells and reduces vascular permeability, while Ang2, which increases in activated vascular endothelial cells, competes with Ang1 and plays a role in inhibiting Ang1-induced stabilization of vascular endothelial cells. Therefore, Ang2 inhibits Ang 1- Tie2 binding and signaling through Ang 1- Tie2, which maintains the stability of vascular endothelial cells in the presence of VEGF, and ultimately promotes angiogenesis in blood vessels, resulting in increased angiogenesis, destabilization of blood vessels, and an increase of vascular permeability. Meanwhile, in addition to its role as an antagonist that induces the inactivity of the Tie2 receptor, since agonist properties that induce the activity of Tie2 receptors have been reported in some specific situations, including lymphatic vessel formation and maintenance, it is believed to have both the function of an antagonist and a weak agonist and perform various functions depending on the situation.

Since the process of angiogenesis is an essential factor in the growth of cancer, there have been attempts to prevent further growth of cancer by inhibiting angiogenesis by inhibiting the Tie2-dependent function of Ang2 as described above, however, these are known to have the function of preventing their role as antagonists by inhibiting the binding of Ang2 and Tie2 in most cases. In addition to antibodies that interfere with the binding of Ang2 to Tie2, recombinant proteins or antibodies that directly bind to the Tie2 receptor and induce phosphorylation and activation have been reported.

Recently, an antibody that binds to Ang2 and so binds to Tie2 to phosphorylate and activate Tie2 through Tie2 clustering has been reported. This does not simply block Ang2 role as an antagonist, but rather acts as an agonist, leading to the possibility of more effective vascular normalization.

[Prior patent literature]

Republic of Korea Patent Publication No. 10-2020-0144536

[Disclosure]

[Technical Problem]

The object of the present invention is to provide an anti-Ang2 antibody or antigen-binding fragment thereof that specifically binds to Ang2 (Angiopoietin-2), an angiogenesis-inducing factor, and binds to the Tie2 receptor together with Ang2 to effectively induce activation of the Tie2 receptor.

Another object of the present invention is to provide a pharmaceutical composition comprising the anti-Ang2 antibody or antigen-binding fragment thereof as an active ingredient.

Another object of the present invention is to provide a pharmaceutical composition for the treatment of diseases associated with neovascularization, increased vascular permeability, and/or decreased normal angiogenesis, comprising the anti-Ang2 antibody or antigen-binding fragment thereof as an active ingredient.

Another object of the present invention is to provide a pharmaceutical composition for preventing or treating cancer comprising the anti-Ang2 antibody or antigen-binding fragment thereof as an active ingredient.

Another object of the present invention is to provide a composition for diagnosing diseases related to Ang2 overexpression, comprising the anti-Ang2 antibody or antigen-binding fragment thereof.

Another object of the present invention is to provide a nucleic acid encoding the antibody or antigen-binding fragment thereof, a vector and host cell comprising the nucleic acid, and a method for producing an anti-Ang2 antibody or antigen-binding fragment thereof using the same.

[Technical Solution]

In order to achieve the above object, the present invention is an antibody or antigen-binding fragment thereof that specifically binds to angiopoietin 2 (Ang2) and induces Tie2 activation, comprising a heavy chain complementarity determining region (CDR) comprising (a) CDRH1 of the amino acid sequence of SEQ ID NO: 2, SEQ ID NO: 10, SEQ ID NO: 18, or SEQ ID NO: 26, CDRH2 of the amino acid sequence of SEQ ID NO: 3, SEQ ID NO: 11, SEQ ID NO: 19, or SEQ ID NO: 27, and CDRH3 of the amino acid sequence of SEQ ID NO: 4, SEQ ID NO: 12, SEQ ID NO: 20, or SEQ ID NO: 28; and (b) a light chain CDR comprising CDRL1 of the amino acid sequence of SEQ ID NO: 5, SEQ ID NO: 13, SEQ ID NO: 21, or SEQ ID NO: 29, CDRL2 of the amino acid sequence of SEQ ID NO: 6, SEQ ID NO: 14, SEQ ID NO: 22, or SEQ ID NO: 30, and CDRL3 of the amino acid sequence of SEQ ID NO: 7, SEQ ID NO: 15, SEQ ID NO: 23, or SEQ ID NO: 31.

In one embodiment of the present invention, the antibody or antigen-binding fragment thereof preferably comprises (a) a heavy chain complementarity determining region (CDR) comprising CDRH1 of the amino acid sequence of SEQ ID NO: 2, CDRH2 of the amino acid sequence of SEQ ID NO: 3, and CDRH3 of SEQ ID NO: 4; and (b) a light chain CDR comprising CDRL1 of the amino acid sequence of SEQ ID NO: 5, CDRL2 of the amino acid sequence of SEQ ID NO: 6, and CDRL3 of the amino acid sequence of SEQ ID NO: 7;

In another embodiment of the invention, the antibody or antigen-binding fragment thereof preferably comprises (a) a heavy chain complementarity determining region (CDR) comprising CDRH1 of the amino acid sequence of SEQ ID NO: 10, CDRH2 of the amino acid sequence of SEQ ID NO: 11, and CDRH3 of SEQ ID NO: 12; and (b) a light chain CDR comprising CDRL1 of the amino acid sequence of SEQ ID NO: 13, CDRL2 of the amino acid sequence of SEQ ID NO: 14, and CDRL3 of the amino acid sequence of SEQ ID NO: 15, but is not limited thereto.

In another embodiment of the invention, the antibody or antigen-binding fragment thereof preferably comprises (a) a heavy chain complementarity determining region (CDR) comprising CDRH1 of the amino acid sequence of SEQ ID NO: 18, CDRH2 of the amino acid sequence of SEQ ID NO: 19, and CDRH3 of SEQ ID NO: 20; and (b) a light chain CDR comprising CDRL1 of the amino acid sequence of SEQ ID NO: 21, CDRL2 of the amino acid sequence of SEQ ID NO: 22, and CDRL3 of the amino acid sequence of SEQ ID NO: 23, but not limited thereto.

In another embodiment of the invention, the antibody or antigen-binding fragment thereof preferably comprises (a) a heavy chain complementarity determining region (CDR) comprising CDRH1 of the amino acid sequence of SEQ ID NO: 26, CDRH2 of the amino acid sequence of SEQ ID NO: 27, and CDRH3 of SEQ ID NO: 28; and (b) a light chain CDR comprising CDRL1 of the amino acid sequence of SEQ ID NO: 29, CDRL2 of the amino acid sequence of SEQ ID NO: 30, and CDRL3 of the amino acid sequence of SEQ ID NO: 31; preferably, but not limited thereto.

In one embodiment of the present invention, the antibody or antigen-binding fragment thereof preferably comprises a heavy chain variable region comprising an amino acid sequence selected from the group consisting of SEQ ID NO: 8, SEQ ID NO: 16, SEQ ID NO: 24, and SEQ ID NO: 32, and a light chain variable region comprising an amino acid sequence selected from the group consisting of SEQ ID NO: 9. , SEQ ID NO: 17, SEQ ID NO: 25, and SEQ ID NO: 33, but is not limited thereto.

In one embodiment of the present invention, the antibody or antigen-binding fragment thereof is preferably, but not limited to, a murine antibody, chimeric antibody, or humanized antibody.

As used herein, "chimeric antibody" comprises a antibody is derived from another species such as an antibody in which the variable region sequence is derived from one species and the variable region sequence is derived from a mouse antibody and the constant region is derived from a human antibody.

As used herein, the term "humanized antibody" comprises antibodies in which CDR sequences derived from the germline of another mammalian species, such as mouse, are grafted onto human framework regions. Additional modifications of framework domain may be made within the human framework sequences as well as within CDR sequences derived from the germline of another mammalian species.

The present invention also provides an isolated nucleic acid encoding the anti-Ang2 antibody or antigen-binding fragment thereof of the present invention.

In one embodiment of the present invention, the antibody or fragment thereof specifically binds to angiopoietin-2, and preferably binds to the Tie2 receptor together with Ang2, but is not limited thereto.

In one embodiment of the present invention, the antigen-binding fragment is preferably selected from the group consisting of scFv, $(scFv)_2$, scFv-Fc, Fab, Fab', and $F(ab')_2$, but is not limited thereto.

The present invention also provides a pharmaceutical composition for the prevention or treatment of diseases associated with Ang2 overexpression, neovascularization, or increased vascular permeability, comprising the anti-Ang2 antibody or antigen-binding fragment thereof of the present invention as an active ingredient.

In one embodiment of the present invention, the disease associated with Ang2 overexpression, neovascularization, or increased vascular permeability is preferably, but not limited to, cancer, cancer metastasis, inflammatory disease, infection, cardiovascular disease, kidney disease, hereditary hemorrhagic telangiectasia, asthma, or edema.

In one embodiment of the present invention, the cancer includes, but is not limited to, carcinoma, lymphoma, blastoma, sarcoma, and leukemia. More specific examples of these cancers include squamous cell carcinoma, lung cancer (including small cell lung cancer, non-small cell lung cancer, adenocarcinoma of the lung, and squamous cell tumor of the lung), cancer of the peritoneum, hepatocellular carcinoma, stomach, or gastrointestinal cancer (including gastrointestinal cancer and gastrointestinal stromal cancer), pancreatic cancer, glioblastoma, cervical cancer, ovarian cancer, hepatocellular cancer, bladder cancer, liver cancer, breast cancer, colon cancer, rectal cancer, endometrial or uterine cancer, salivary gland carcinoma, kidney cancer or kidney cell cancer, liver cancer, prostate cancer, and vulvar cancer. thyroid cancer, liver carcinoma and various types of head and neck cancer, melanoma, superficial spreading melanoma, malignant melanoma, acral lentigo melanoma, nodular melanoma as well as B-cell lymphoma (including low grade/follicular non-Hodgkin lymphoma (NHL); Small lymphocytic (SL) NHL; intermediate/follicular NHL; intermediate diffuse NHL; high-grade immunoblastic NHL; high-grade lymphoblastic NHL; high-grade small-cell NHL; large tumor NHL; mantle cell lymphoma; AIDS-related lymphoma; Waldenstrom macroglobulin); chronic lymphocytic leukemia (CLL); Acute lymphocytic leukemia (ALL); Hairy cell leukemia; chronic myeloblastic leukemia; post-transplant lymphoproliferative disease (PTLD), as well as nevi, edema (such as that associated with brain cancer), and abnormal vascular proliferations such as Meigs syndrome, but are not limited to.

In addition, the present invention provides a pharmaceutical composition for the prevention or treatment of diseases associated with reduced normal blood vessel formation, comprising the Ang2 antibody or antigen-binding fragment thereof of the present invention as an active ingredient.

In one embodiment of the present invention, the disease associated with reduced normal blood vessel formation is preferably myocardial infarction, angina pectoris, cerebral infarction, stroke, Buerger's disease, avascular necrosis, foot ulcer, or erectile dysfunction, but is not limited thereto.

Additionally, the present invention provides a pharmaceutical composition for preventing or treating disease Alzheimer's disease; inflammatory autoimmune diseases such as rheumatoid arthritis, multiple sclerosis, or psoriasis; eye diseases such as age-related macular degeneration (AMD), diabetic macular edema (DME), diabetic retinopathy (DR), or glaucoma; coronavirus infectious diseases such as COVID-19 comprising the anti-Ang2 antibody or antigen-binding fragment thereof of the present invention as an active ingredient.

In addition, the present invention provides combination therapy composition for use in treatment for cancer comprising administering the anti-Ang2 antibody or antigen-binding fragment thereof of the present invention simultaneously, separately, or sequentially with irradiation, a chemotherapy agent, a cytotoxic agent, and/or an immunotoxic agent.

In one embodiment of the present invention, the chemotherapeutic agent, cytotoxic agent and/or immunotoxic agent is preferably temozolomide, cisplatin, oxaliplatin, carboplatin, 5-FU, darcabarzine, procarbazine, Vinblastine, vincristine, irinotecan, taxol, paclitaxel, docetaxel, gemcitabine, Gleevec, Iressa, Tarceva and Nexavar, Herceptin, bevacizumab, cetuximab, nimotuzumab, sorafenib, sunitinib and ZD6474 (ZACTIMATM), more preferably one or more selected from temozolomide, cisplatin, oxaliplatin, vinblastine, taxol, gemcitabine, Gleevec and Iressa, but is not limited thereto.

The present invention is described below.

Unless otherwise defined, all technical and scientific terms used in the present invention have the same meaning as commonly understood by a person skilled in the art to which the present invention pertains. In general, the nomenclature used herein is well known and commonly used in the art.

The present invention provides an antibody or antigen-binding fragment thereof with a dual function in which the antibody that specifically binds to Ang2 but does not inhibit the binding of Ang2 to the Tie2 receptor and forms a complex (antibody/Ang2/Tie2) with Ang2 and the Tie2 receptor binds to the Tie2 receptor together with Ang2 and activates the Tie2 receptor like Ang1 to induce Tie2 downstream signaling and stabilization of vascular endothelial cells.

In addition, in addition to the effect of activating the Tie2 receptor like Ang1 by binding to the Tie2 receptor together with Ang2, anti-Ang2 antibody that has a more enhanced cancer cell growth inhibition and/or cancer metastasis inhibition effect by inhibiting the binding of other proteins involved in cancer cell growth and/or cancer metastasis, such as integrins and Ang2 and this effect is also maintained in cells that do not express Tie2 and their medicinal uses are provided.

In one embodiment of the present invention, the heavy chain complementarity determining region, the light chain complementarity determining region, or a combination thereof of the anti-Ang2 antibody described above; or a polypeptide molecule comprising a heavy chain variable region, a light chain variable region, or a combination thereof is provided. The polypeptide molecule can function as a precursor or component of an antagonist for Ang2 as well as for the construction of antibodies or antigen-binding fragments thereof. For example, the polypeptide molecule may serve as an Ang2 antigen binding site and may be included as a component of a protein framework (e.g., peptibody, nanobody, etc.) with a structure similar to an antibody, a dual-specific antibody, a multi-specific antibody, etc.

The term "antagonist" is intended to include any molecule that partially or completely blocks, inhibits or neutralizes one or more of the biological activities of a target (e.g., Ang2).

The term "peptibody (peptide + antibody)" refers to a fusion protein in which a peptide and all or part of a constant region such as the Fc portion of an antibody are fused, and the peptide serves as an antigen binding site (heavy chain and/or light chain CDR or variable region), with a framework and function similar to that of an antibody.

The term "nanobody", also called a single-domain antibody, refers to an antibody fragment comprising a single variable domain of an antibody in monomeric form and selectively binds to a specific antigen, similar to a complete antibody. The molecular weight of nanobodies is typically about 12 kDa to about 15 kDa, which is very small compared to the typical molecular weight (about 150 kDa to about 160 kDa) of a complete antibody (including two heavy and two light chains), and in some cases, it is smaller than the Fab fragment or scFv fragment.

The term "bispecific antibody" or "multi-specific antibody" refers to an antibody that recognizes and/or binds to two (bispecific antibodies) or more (multi-specific antibodies) different antigens, or recognizes and/or binds to different regions of the same antigen and the antigen binding site of either the bispecific antibody or the multi-specific antibody may include the polypeptide.

In an embodiment, the polypeptide molecule may include a polypeptide comprising:

one or more selected from the group consisting of a polypeptide comprising the amino acid sequence of SEQ ID NO: 2, SEQ ID NO: 10, SEQ ID NO: 18, or 26, the amino acid sequence of SEQ ID NO: 3, SEQ ID NO: 11, SEQ ID NO: 19, or SEQ ID NO: 27 and the amino acid sequence of SEQ ID NO: 4, SEQ ID NO: 12, SEQ ID NO: 20, or SEQ ID NO: 28; and

one or more selected from the group consisting of polypeptides comprising the amino acid sequence of SEQ ID NO: 7, SEQ ID NO: 15, SEQ ID NO: 23, or SEQ ID NO: 31, amino acid sequence of SEQ ID NO: 5, SEQ ID NO: 13, SEQ ID NO: 21, or SEQ ID NO: 29, the amino acid sequence of SEQ ID NO: 6, SEQ ID NO: 14, SEQ ID NO: 22, or SEQ ID NO: 30, or a combination thereof.

In an embodiment, the polypeptide molecule may include the amino acid sequence of SEQ ID NO: 8, SEQ ID NO: 16, SEQ ID NO: 24, or SEQ ID NO: 32, and the amino acid sequence of SEQ ID NO: 9, SEQ ID NO: 17, SEQ ID NO: 25, or SEQ ID NO: 33, or a combination thereof.

The terms "Kabat number", "Kabat definition", and "Kabat labeling" are used interchangeably herein. These terms, recognized in the technical field to which the present invention pertains, refer to a numbering system for amino acid residues that exhibit greater diversity (i.e., high variability) than other amino acid residues in the variable regions of the heavy and light chains of an antibody or in the corresponding region of the antigen binding site. (Kabat et al. (1971) Ann. NY Acad, Sci) 190:382-391, Kabat, E. A. et al. (1991) Sequences of proteins of immunological interest, no. 5th Edition, Department of Health and Human Services, National Institutes of Health, Publication No. 91-3242).

As described above, the bispecific antibody or multi-specific antibody is an antibody that simultaneously recognizes and/or binds two or more types of antigens, including each antigen-binding site for two or more different antigens. As such, one of the antigen binding sites may include the polypeptide molecule described above. Specifically, the polypeptide molecule serving as the Ang2 antigen binding site may form a dimer or multimer with an antigen binding site for another antigen to constitute a bispecific antibody or multi-specific antibody. Accordingly, in one embodiment, a bispecific or multi-specific antibody is provided comprising the above polypeptide molecule as an Ang2 antigen binding site.

In another example, one or more polypeptide molecules described above or a repeat of the polypeptide molecules repeatedly linked by a linker (hereinafter referred to as 'first peptide') and a polypeptide that performs a structural function (hereinafter referred to as 'second peptide'; for example, including one or more (e.g., 1 to 5, or 2 to 4) peptide complexes comprising the constant region of the heavy or light chain of an antibody (IgG, IgA, IgE, IgD, IgM, etc.) or the Fc fragment of an antibody)) and the one or more peptide complexes are combined in a second peptide(for example Fc fragment) to provide a protein framework with a multimeric structure.

In the present invention, antibodies comprise animal-derived antibodies, chimeric antibodies, humanized antibodies, and human antibodies. Since animal-derived antibodies produced by immunizing an immunized animal with a desired antigen can generally cause immune rejection when administered to humans for therapeutic purposes, chimeric antibodies were developed to suppress this immune rejection. A chimeric antibody is one in which the constant region of an animal-derived antibody, which causes an anti-isotype reaction, is replaced with the constant region of a human antibody using genetic engineering methods. Chimeric antibodies have significantly improved anti-isotype responses compared to animal-derived antibodies, but animal-derived amino acids still exist in the variable region, resulting in potential side effects on anti-idiotypic responses. Humanized antibodies were developed to improve these side effects. It is produced by transplanting the CDR (complementary determining regions) region, which plays an important role in antigen binding, among the variable regions of a chimeric antibody, into the human antibody framework.

The most important thing in CDR grafting technology to produce humanized antibodies is to select an optimized human antibody that can best accept the CDR region of an animal-derived antibody, for this purpose, the use of antibody databases, crystal structure analysis, and molecular modeling technology are used. However, even when the CDR region of an animal-derived antibody is transplanted onto an optimized human antibody framework, there are cases where amino acids that affect antigen binding are present in the framework of the animal-derived antibody, so antigen-binding ability is not preserved in many cases. Therefore, the application of additional antibody engineering technology to restore antigen binding ability is essential.

According to one embodiment, the antibody may be a mouse-derived antibody, a mouse-human chimeric antibody, a humanized antibody, or a human antibody.

In the present invention, "antibody" refers to a substance produced by stimulation of an antigen within the immune system, and its type is not particularly limited. Antibodies have recently been widely used as disease treatments. Antibodies are very stable not only in vitro but also in vivo and have a long half-life, making them advantageous for mass expression and production. In addition, antibodies inherently have a dimer structure, so their avidity is very high.

A complete antibody has a structure of two full-length light chains and two full-length heavy chains, and each light chain is connected to the heavy chain by a disulfide bond. The constant region of an antibody is divided into a heavy chain constant region and a light chain constant region, and the heavy chain constant region has gamma ($\gamma$), mu ($\mu$), alpha ($\alpha$), delta ($\delta$), and epsilon ($\varepsilon$) types, and subclasses has gamma 1 ($\gamma$1), gamma 2 ($\gamma$2), gamma 3 ($\gamma$3), gamma 4 ($\gamma$4), alpha 1 ($\alpha$1), and alpha 2 ($\alpha$2). The constant region of the light chain has kappa ($\kappa$) and lambda ($\lambda$) types.

The term "heavy chain" is interpreted as both the full-length heavy chain and fragments comprising a hinge and a variable region domain VH which comprise an amino acid sequence with sufficient variable region sequence to confer specificity to the antigen and three constant region domains CH1, CH2 and CH3. In addition, the term "light chain" is interpreted as both a full-length light chain and fragments thereof comprising the variable region domain VL which comprise an amino acid sequence with sufficient variable region sequence to confer specificity to the antigen and the constant region domain CL.

The term "complementarity determining region (CDR)" refers to the amino acid sequence of the hypervariable region of the heavy and light chains of immunoglobulins. The heavy and light chains may each comprise three CDRs (CDRH1, CDRH2, CDRH3 and CDRL1, CDRL2, CDRL3). The CDR may provide key contact residues for the antibody to bind to the antigen or epitope. Meanwhile, in the present specification, the term "specifically binds" or "specifically recognizes" has the same meaning commonly known to those skilled in the art and it means that antigens and antibodies specifically interact to produce an immune response.

The antigen-binding fragment of the antibody provided in the present invention may be a fragment comprising one or more of the above complementarity-determining regions.

The term "antigen-binding fragment" refers to a fragment of the entire immunoglobulin structure, a portion of a polypeptide comprising a portion to which an antigen can bind. For example, it may be scFv, (scFv)$_2$, scFv-Fc, Fab, Fab' or F(ab')$_2$, but is not limited thereto.

Among the above antigen-binding fragments, an Fab, which is a structure having the light chain and heavy chain variable regions, the light chain constant region, and the heavy chain first constant region (CH1), has one antigen binding site. An Fab' differs from the Fab in that the Fab' has a hinge region including at least one cysteine residue at the C-terminal of the heavy chain CH1 domain. An F(ab')$_2$ is produced when cysteine residues at the hinge region of Fab' are joined by a disulfide bond. An Fv is a minimal antibody fragment, having only heavy chain variable regions and light chain variable regions, and a recombinant technique for producing the Fv fragment is well known in the art. In a two-chain Fv fragment, the heavy chain variable domains are associated with the light chain variable domains via a noncovalent bond. A single-chain Fv fragment has a structure in which a heavy chain variable domain and a light chain variable domain are covalently joined to each other via a covalent bond or directly at the C-terminus, so that it can form a dimer as in a two-chain Fv fragment. The peptide linker may be composed of 1 to 100 amino acid residues, or 2 to 50 amino acid residues, with no limitations imposed on the kind of the amino acid residues. Amino acid sequences suitable for use in the peptide linker may be those well known in the art. The antigen-binding fragment may be obtained using a protease (for example, a whole antibody can be digested with papain to obtain Fab fragments, or can be digested with pepsin to obtain F(ab')$_2$ fragments), or may be prepared by a genetic recombinant technique.

The term "hinge region" refers to a region included in the heavy chains of an antibody, which is present between the CH1 and CH2 regions, and provides flexibility to the antigen binding site in the antibody. For example, the hinge may be derived from a human antibody and particularly, it may be derived from IgA, IgE, IgD, IgM, or IgG, for example, IgG1, IgG2, IgG 3, or IgG4.

When an animal-derived antibody goes through a chimerization process, an animal-derived IgG1 hinge is replaced with a human IgG1 hinge, but a length of the animal-derived IgG1 hinge is shorter than the human IgG1 hinge, and disulfide bonds between two heavy chains are reduced from 3 to 2. Thus, rigidity of the hinges may have different effects. Therefore, modification of a hinge region can increase an antigen binding efficiency of a humanized antibody. Methods of deleting, inserting, or substituting an amino acid for modifying amino acid sequences of the hinge region are well known in the art.

Portions except the variable regions of the anti-Ang2 antibody may be constant regions derived from a human antibody and particularly, they may be derived from IgA, IgD, IgE, IgD, IgM, or IgG, for example, IgG1, IgG2, IgG 3, or IgG4.

The anti-Ang2 antibody may be a monoclonal antibody. The monoclonal antibody may be prepared by methods well known in the art. For example, it may be prepared using a phage display technique. Alternately, the Ang2 antibody may be prepared into a mouse-derived monoclonal antibody by methods set forth in the paper written by Schwaber, et al (Schwaber, J and Cohen, E. P., "Human x Mouse Somatic Cell Hybrid Clones Secreting Immunoglobulins of Both Parental Types," Nature, 244 (1973), 444-447).

Meanwhile, individual monoclonal antibodies may be screened using a typical ELISA (Enzyme-Linked ImmunoSorbent Assay) format, based on the binding potential with Ang2. Inhibitory activities can be verified through functional analysis such as competitive ELISA for verifying the molecular interaction of binding assemblies or functional analysis such as a cell-based assay. Then, regarding monoclonal antibody members selected on the basis of their strong inhibitory activities, their affinities (Kd values) to Ang2 may be each verified.

The rest portions except the antigen binding portions of the finally selected antibodies may be prepared as not only human immunoglobulin antibodies but also humanized antibodies. Preparation of humanized antibodies is well known in the art (Almagro, J. C. and Fransson, J., "Humanization of antibodies," Frontiers in Bioscience, 13(2008), 1619-1633).

Another embodiment provides a pharmaceutical composition for inhibiting angiogenesis comprising the anti-Ang2 antibody or an antigen-binding fragment thereof as an active ingredient. Another embodiment provides a method for inhibiting angiogenesis comprising administering a pharmaceutically effective amount of the anti-Ang2 antibody or an antigen-binding fragment thereof to a subject who may need inhibiting angiogenesis. The angiogenesis inhibition method may further include a step of identifying a subject who needs the inhibition of angiogenesis, prior to the administration step.

Another embodiment provides a pharmaceutical composition for reducing vascular permeability comprising the anti-Ang2 antibody or an antigen-binding fragment thereof as an active ingredient. Another embodiment provides a method for reducing vascular permeability including administering a pharmaceutically effective amount of the anti-Ang2 antibody or an antigen-binding fragment thereof to a subject who may need the reduction of vascular permeability. The vascular permeability reduction method may further include a step of identifying a subject who needs the reduction of vascular permeability, prior to the administration step.

Another embodiment provides a pharmaceutical composition for preventing and/or treating a disease related to Ang2 overexpression, angiogenesis, and/or vascular permeability increase comprising the anti-Ang2 antibody or an antigen-binding fragment thereof as an active ingredient. Another embodiment provides a method for preventing and/or treating a disease related to Ang2 overexpression, angiogenesis, and/or vascular permeability increase comprising administering a pharmaceutically effective amount of the anti-Ang2 antibody or an antigen-binding fragment thereof to a subject who may need preventing and/or treating the disease related to Ang2 overexpression, angiogenesis, and/or vascular permeability increase. The prevention and/or treatment method may further comprise a step of identifying a subject who need preventing and/or treating a disease related to Ang2 overexpression, angiogenesis, and/or vascular permeability increase, prior to the administration step.

Another embodiment provides a pharmaceutical composition for inducing normal blood vessel formation comprising the anti-Ang2 antibody or an antigen-binding fragment thereof as an active ingredient. Another embodiment provides a method for inducing normal blood vessel formation, including administering a pharmaceutically effective amount of the anti-Ang2 antibody or an antigen-binding fragment thereof to a subject who may need inducing normal blood vessel formation. The normal blood vessel formation induction method may further include a step of identifying a subject who needs inducing normal blood vessel formation, prior to the administration step.

Another embodiment provides a pharmaceutical composition for preventing and/or treating a disease related to normal blood vessel formation decrease comprising the anti-Ang2 antibody or an antigen-binding fragment thereof as an active ingredient. Another embodiment provides a method for preventing and/or treating a disease related to normal blood vessel formation decrease comprising administering a pharmaceutically effective amount of the anti-Ang2 antibody or an antigen-binding fragment thereof to a subject who may need preventing and/or treating the disease related to normal blood vessel formation decrease. The prevention and/or treatment method may further comprise a step of identifying a subject who need preventing and/or treating a disease related to normal blood vessel formation decrease, prior to the administration step.

Another embodiment provides a composition for diagnosing a disease related to angiogenesis, and/or vascular permeability increase and/or normal blood vessel formation decrease, comprising the antibody or an antigen binding fragment thereof.

The pharmaceutical compositions may further comprise a pharmaceutically acceptable carrier, and the carrier may be those commonly used in the formulation of drugs, which may be one or more selected from the group consisting of lactose, dextrose, sucrose, sorbitol, mannitol, starch, gum acacia, calcium phosphate, alginates, gelatin, calcium silicate, micro-crystalline cellulose, polyvinylpyrrolidone, cellulose, water, syrup, methyl cellulose, methylhydroxy benzoate, propylhydroxy benzoate, talc, magnesium stearate, and mineral oil, but is not limited thereto. The pharmaceutical compositions may further include one or more selected from the group consisting of a diluent, an excipient, a lubricant, a wetting agent, a sweetener, a flavor enhancer, an emulsifying agent, a suspension agent, and a preservative.

Pharmaceutically effective amounts of the pharmaceutical compositions, or the antibody or the antigen-binding fragment thereof may be administered orally or parenterally. The parenteral administration may include intravenous injection, subcutaneous injection, muscular injection, intraperitoneal injection, endothelial administration, local administration, intranasal administration, intrapulmonary administration, and rectal administration. Since oral administration leads to digestion of proteins or peptides, an active ingredient in the compositions for oral administration must be coated or formulated to prevent digestion in stomach. In addition, the composition may be administered using an optional device that enables an active substance to be delivered to target cells.

The content of the anti-Ang2 antibody or an antigen-binding fragment thereof in the pharmaceutical compositions may be prescribed in a variety of ways, depending on factors such as formulation methods, administration methods, age of subjects, body weight, gender, pathologic conditions, diets, administration time, administration interval, administration route, excretion speed, and reaction sensitivity. For example, a daily dosage of the anti-Ang2 antibody or an antigen-binding fragment thereof may be within the range of 0.001 to 1000mg/kg, particularly 0.01 to 100mg/kg, and more particularly 0.1 to 50mg/kg, but is not limited thereto. The daily dosage may be formulated into a single formulation in a unit dosage form or formulated in suitably divided dosage forms, or it may be manufactured to be contained in a multiple dosage container. The term "pharmaceutically effective amount" as used herein refers to a content or dose of an active ingredient capable of showing desirable pharmacological effects and it may be determined in a variety of ways, depending on factors such as formulation methods, administration methods, age of subjects, body weight, gender, pathologic conditions, diets, administration time, administration interval, administration route, excretion speed, and reaction sensitivity.

The pharmaceutical compositions may be formulated into a form of a solution in oil or an aqueous medium, a suspension, syrup, an emulsifying solution, an extract, powder, granules, a tablet, or a capsule, and may further include a dispersing or a stabilizing agent for the formulation.

In particular, the pharmaceutical compositions comprising the anti-Ang2 antibody or an antigen-binding fragment thereof may be formulated into an immunoliposome since it comprises an antibody or an antigen-binding fragment. A liposome comprising an antibody may be prepared using any methods widely known in the art. The immunoliposome may be a lipid composition comprising phosphatidylcholine, cholesterol, and polyethyleneglycolderivatized phosphatidylethanolamine, and may be prepared by a reverse phase evaporation method. For example, Fab' fragments of an antibody may be conjugated to the liposome through a disulfide-exchange reaction.

Meanwhile, as the anti-Ang2 antibody or an antigen-binding fragment thereof specifically binds to Ang2, this can be used to detect Ang2, and the presence of the overexpression of Ang2 can be verified through it. Accordingly, another embodiment provides a composition for detecting Ang2 and a composition for diagnosing a disease related to Ang2 overexpression comprising the anti-Ang2 antibody or an antigen-binding fragment thereof.

[Advantageous Effects]

The present invention proposes an antibody that inhibits Ang2 and simultaneously activates the Tie2 receptor to promote downstream signal transduction, thereby providing a new method to inhibit angiogenesis caused by Ang2 and reduce vascular permeability. In addition, the antibody proposed in the present invention is expected to have applicability in the diagnosis and treatment of diseases other than cancer related to abnormal angiogenesis and/or diseases caused by increased vascular permeability. The antibody can be used in combination therapy with chemical drugs and other anticancer treatments, and is expected to be used for antibody fragments, bi- or multi-specific antibodies, protein framework, etc. using Ang2-specific recognition.

[Description of Drawings]

Figure 1 is a western blot result showing that anti-Ang2 antibody phosphorylates AKT and ERK 42/44 in HUVEC cells together with Ang2.

Figure 2 is an ELISA result showing that anti-Ang2 antibody induces AKT phosphorylation in HUVEC cells together with Ang2 in an antibody concentrationdependent manner. In order from the curve with the highest value in the graph of the figure, the highest clone is 1D3, then 2C8, then 1A9, then 1H10, and the clone with the lowest value is the control 67 clone.

Figure 3 shows the binding affinity of anti-Ang2 antibody to human Ang2 and mouse Ang2 analyzed through ELISA.

Figure 4 is an ELISA result showing that anti-Ang2 antibody binds to Ang2 and Tie2 to form a complex.

Figure 5 is an ELISA result showing that anti-Ang2 antibody 2C8 does not interfere with the binding of Ang2 and Tie2.

Figure 6 shows that anti-Ang2 antibody 2C8 inhibits tumor growth in the LLC lung cancer cell tumor model (error bar: standard error of mean).

Figure 7 shows that the combined administration of anti-Ang2 antibody 2C8 and cisplatin has a significantly better tumor growth inhibition effect than each administration alone in the LLC lung cancer cell tumor model (error bar: standard error of mean).

Figure 8 shows that the combined administration of anti-Ang2 antibody 2C8 and 5FU has a significantly superior tumor growth inhibition effect compared to each administration alone in the MC38 colon cancer cell tumor model (error bar: standard error of mean).

[Mode for Invention]

Hereinafter, the present invention will be described in more detail through examples, but these are merely illustrative and are not intended to limit the scope of the present invention. It is obvious to those skilled in the art that the embodiments described below can be modified without departing from the essential gist of the invention.

[Example]

## Example 1: **Preparation and screening of anti-Ang2 antibody**

An antibody that specifically binds to human Ang2 (SEQ ID NO: 1) as an antigen was ordered from AbClone (Korea), and the antibody of the present invention was manufactured by Kohler and Milstein, Eur. J Immunol. 6, 511 (1976).

In summary, human Ang2 mixed with an adjuvant (Sigma) as an antigen was injected twice into mice, and antibody production was confirmed using ELISA. After two immunizations, the antibody titer (1:5000) increased, so the spleens were removed from the immunized mice, and B lymphocytes were isolated and fused with sp2/0 cells. The fused cells were cultured in a medium (HAT medium) supplemented with hypoxantin, aminopterine, and thymidine, and cells (hybridoma) in which B lymphocytes and sp2/0 cells were fused were selectively selected. The process (cloning) of separating positive and negative cells from the obtained hybridoma cells using the serial dilution method was repeated to prepare monoclonal cells that produce antibodies that react to the antigen.

The antibody-producing hybridoma cells obtained above were cultured in DMEM (Dulbeco's Modified Eagle's Mediaum) containing 10% (v/v) FBS at 37°C and 5% $CO_2$, and then centrifuged to obtain antibody-producing cells. The culture medium in which the antibodies were secreted was separated, and the antibodies were purified using an affinity column (Protein A/G agarose column, Protein A/G (GenDEPOT)).

## Example 2: **Western blot induction of activation of Tie2 signaling by anti-Ang2 antibody**

Ang2 binds to the Tie2 receptor expressed on vascular endothelial cells and acts as a weak agonist or antagonist. Since the anti-Ang2 antibody developed in the present invention binds to Ang2 and forms a complex with Ang2-Tie2, thereby activating the Tie2 receptor and promoting downstream signaling, ERK and AKT phosphorylation experiments were performed to analyze the effect of anti-Ang2 antibodies on Tie2 downstream signaling using a cell-based assay. To compare the degree of activation of Tie2 downstream signaling, the same test was performed on the group treated with Ang2 (Sino Biological) and another Ang2 control antibody (Korean Patent Publication No. 10-2015-0136031).

Specifically, HUVEC (Lonza) cells ($2 \times 10^5$) were cultured at 37°C using EGM-2 (Lonza) medium, and when 80-90% confluency was observed, they were changed to 0.5% FBS Basal media (Lonza) for 16 to 24 days. Cultured at 37°C for 1 hour. 60 nM of anti-Ang2 antibody was mixed with 40 nM of Ang2 protein (Sino Biological) and left for 30 minutes. Then, the cultured cells were treated and incubated for another 10 minutes. For comparison, groups treated with 40 nM Ang2 and 40 nM Ang2 + 60 nM anti-Ang2 control antibody were prepared. After washing the cells using PBS, lysis buffer (Ripa buffer, 0.15M Sodium chloride, 1% Triton X-100, 1% Sodium deoxycholate, 0.1% SDS, 50mM Tris-HCl, pH 7.5, and 2mM EDTA), centrifuged at 13,000 rpm for 15 minutes to recover the supernatant, and obtain cell lysate.

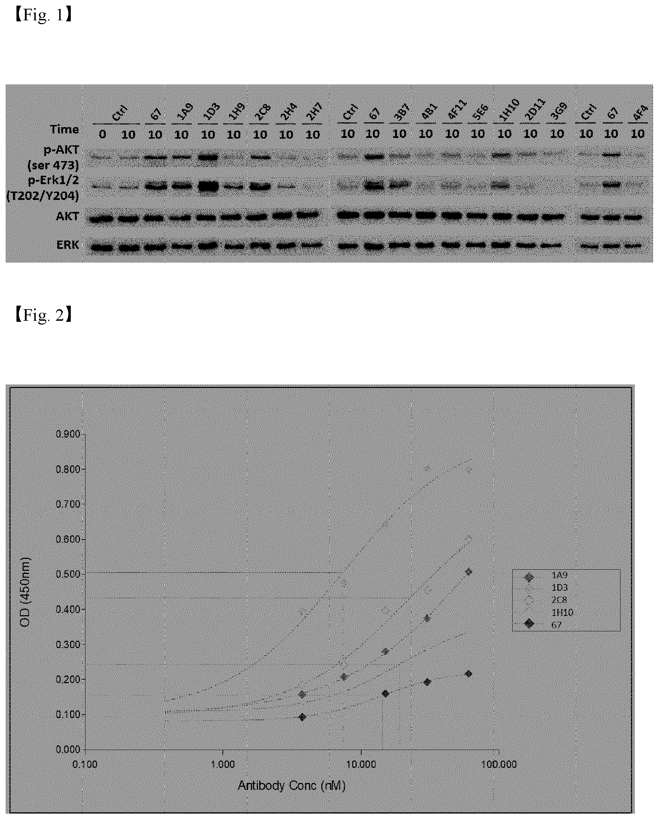

Sample buffer (Biosesang) mixed with a reducing agent was added to 35 $\mu$g of the cell lysate, boiled at 95°C for 5 minutes, electrophoresed on a 10% Tris-Glycine gel, and transferred to a nitrocellulose membrane (GVS). To check the phosphorylation of Akt and ERK 42/44, which are involved in downstream signaling of the Tie2 receptor, the above blot was blocked for 1 hour with PBST mixed with 5% (v/v) skim milk (Seoul Milk) and anti-phosphorylated Akt antibody (Cell signaling), anti-AKT antibody (Cell signaling), anti-phosphorylated ERK 42/44 antibody (Cell signaling), and anti-ERK 42/44 antibody (Santa Cruz) were treated. The obtained results are shown in Figure 1.

As shown in Figure 1, 1A9, 1D3, 2C8, and 1H10 clones showed AKT and ERK activation signals compared to the negative control group.

Example 3: **ELISA for induction of activation of Tie2 downstream signaling by anti-Ang2 antibody**

To quantitatively analyze the effect of anti-Ang2 antibodies on Tie2 downstream signaling, the level of AKT phosphorylation was measured using ELISA.

HUVEC (Lonza) cells ($2 \times 10^5$) were cultured at 37°C using EGM-2 (Lonza) medium, and when 80-90% confluency was observed, they were changed to 0.5% FBS Basal media (Lonza) and cultured at 37°C for 16 to 24 hours. 60 nM of anti-Ang2 antibody was mixed with 40 nM of Ang2 protein (Sino Biological) and left for 30 minutes. Then, the cultured cells were treated and incubated for another 10 minutes. For comparison, groups treated with 40 nM of Ang2, 40 nM of Ang2 + 60 nM of anti-Ang2 control antibody (h10D6-OPTI-67, US 10934350) were prepared. The cells were washed with PBS, treated with the lysis buffer, centrifuged at 13,000 rpm for 15 minutes, and the supernatant was recovered to obtain a cell lysate.

For ELISA, 50 $\mu$L of PathScan®Phospho-Akt1 (Ser473) Sandwich ELISA Antibody Pair (Cell signaling) capture antibody diluted 1:100 was added to a 96 well ps half area plate (Greiner cio-one) and coated. Then, the plate was washed four times with PBST (Phosphate Buffer Saline containing 0.05% (v/v) Tween-20) and then blocked with PBST containing 1% (v/v) skim milk at 37°C for 2 days. Afterwards, the plate was washed four times with PBST containing 0.05% Tween-20, and then cell lysate was added and allowed to bind to the capture antibody for 2 hours at 37°C. After washing the plate 4 times with PBST, the secondary antibody diluted 1:1000 in 1% skim milk was allowed to bind at 37°C for 1 hour and then washed 4 times with PBST. Finally, 50 $\mu$L of TMB substrate (Kementec) was added to the plate to induce a color reaction for 10 minutes. Afterwards, 50 $\mu$L of Stop solution was added to stop the reaction, and the OD450 value was measured on a plate reader (BioTek).

The obtained results are shown in Figure 2.

As shown in Figure 2, the monoclonal antibodies 1A9, 1D3, 2C8, and 1H10 of the present invention were confirmed to induce significantly stronger Tie2 downstream signal compared to previously known control antibodies.

**Example 4: Gene cloning of mouse anti-Ang2 antibody**

RNA was isolated from the hybridoma obtained in Example 1 using the AccuPrep Universal RNA extraction kit (Bioneer). And using this as a template, cDNA synthesis and cloning were performed according to previously known methods (Meyer L et al., "A simplified workflow for monoclonal antibody sequencing" 2019, PLoS ONE). cDNA synthesis was performed using the SuperiorScript III cDNA Synthesis kit (Enzynomics), and the synthesized cDNA was PCR amplified and cloned into a vector using the TOPcloner Blunt core kit (Enzynomics). DNA sequence analysis was performed to obtain base and amino acid sequences encoding the CDR, heavy chain variable region, and light chain variable region of each antibody (Tables 1 to 8).

[TABLE 1]

| Domain | CDR | CDR sequence | Sequence number |
|---|---|---|---|
| Heavy chain | CDRH1-Kabat | DYWIG | SEQ ID NO: 2 |
| | CDRH2-Kabat | DIYPGGGYTNCNEKFKG | SEQ ID NO: 3 |
| | CDRH3-Kabat | SDYRNDEGFAD | SEQ ID NO: 4 |
| Light chain | CDRL1-Kabat | SASSSVSSSYLH | SEQ ID NO: 5 |
| | CDRL2-Kabat | RTSNLAS | SEQ ID NO: 6 |
| | CDRL3-Kabat | QQWSGYPYT | SEQ ID NO: 7 |
| Table 1 is the CDR sequence of mouse anti-Ang2 antibody 1A9. | | | |

[TABLE 2]

| Domain | Variable region sequence | Sequence number |
|---|---|---|
| Heavy chain | QVQLQQSGAELVRPGTSVKMSCKAAGYTFTDYWI GWVKQRPGHGLEWIGDIYPGGGYTNCNEKFKGKA TLTADTSSSTAYMQLSSLTSEDSAIYYCSRSDYRND EGFADWGQGTLVTVSAAK | SEQ ID NO: 8 |
| Light chain | ENVLTQSPAIMTASLGQKVTMTCSASSSVSSSYLH WYQQKSGASPKPLIHRTSNLASGVPARFSGSGSGTS YSLTISSVEAEDDATYYCQQWSGYPYTFGGGTKLEI KRADAAPTVS | SEQ ID NO: 9 |
| Table 2 is the variable region sequence of mouse anti-Ang2 antibody 1A9. | | |

[TABLE 3]

| Domain | CDR | CDR sequence | Sequence number |
|---|---|---|---|
| Heavy chain | CDRH1-Kabat | NYWIG | SEQ ID NO: 10 |
| | CDRH2-Kabat | DIYPGGGYTNYNEKFKG | SEQ ID NO: 11 |
| | CDRH3-Kabat | SDYRDDEGFAY | SEQ ID NO: 12 |
| Light chain | CDRL1-Kabat | SASSSVSSSYLH | SEQ ID NO: 13 |
| | CDRL2-Kabat | RTSNLAS | SEQ ID NO: 14 |
| | CDRL3-Kabat | QQWSGYPYT | SEQ ID NO: 15 |
| Table 3 shows the CDR sequence of mouse anti-Ang2 antibody 2C8. | | | |

[TABLE 4]

| Domain | Variable region sequence | Sequence number |
|---|---|---|
| Heavy chain | QVHLQQSGAELVRPGTSVKMSCKAAGYTFTNYWI GWVKQRPGHGLEWIGDIYPGGGYTNYNEKFKGKA TLTADTSSSTAYMQLSSLTSEDSAIYYCSRSDYRDD EGFAYWGQGTLVTVSAAKTTPPKLYPLA | SEQ ID NO: 16 |
| Light chain | ENVLTQSPAIMAASLGQKVTMTCSASSSVSSSYLH WYQQKSGASPKPLIHRTSNLASGVPARFSGSGSGTS YSLTISTVEAEDDATYYCQQWSGYPYTFGGGTKLEI KRADAAPTVSAAASLS | SEQ ID NO: 17 |
| Table 4 shows the variable region sequence of mouse anti-Ang2 antibody 2C8. | | |

[TABLE 5]

| Domain | CDR | CDR sequence | Sequence number |
|---|---|---|---|
| Heavy chain | CDRH1-Kabat | SDYGWN | SEQ ID NO: 18 |
| | CDRH2-Kabat | YISYSGTTSYNPSLKS | SEQ ID NO: 19 |
| | CDRH3-Kabat | SEGTGFYAMDY | SEQ ID NO: 20 |
| Light chain | CDRL1-Kabat | KASQSVSNDVA | SEQ ID NO: 21 |
| | CDRL2-Kabat | YASNRYT | SEQ ID NO: 22 |
| | CDRL3-Kabat | QQDYSSPT | SEQ ID NO: 23 |
| Table 5 shows the CDR sequence of mouse anti-Ang2 antibody 1D3. | | | |

[TABLE 6]

| Domain | Variable region sequence | Sequence number |
|---|---|---|
| Heavy chain | DVQLQESGPGLVKPSQSLSLTCTVTGYSITSDYGWN WIRQFPGNKLEWMGYISYSGTTSYNPSLKSRISITRD TSKNQFFLQLNSVTTEDTATYYCARSEGTGFYAMD YWGQGTSVTVSSAKTTPPKLY | SEQ ID NO: 24 |
| Light chain | SIVMTQTPKFLLVSAGDRVTIICKASQSVSNDVAWY QQKPGQSPKLLIYYASNRYTGVPDRFTGSGYGTDFT FTISTVQAEDLAVYFCQQDYSSPTFGGGTKLEIKRA | SEQ ID NO: 25 |
| Table 6 shows the variable region sequence of mouse anti-Ang2 antibody 1D3. | | |

[TABLE 7]

| Domain | CDR | CDR sequence | Sequence number |
|---|---|---|---|
| Heavy chain | CDRH1-Kabat | NYWIG | SEQ ID NO: 26 |
| | CDRH2-Kabat | IYPGGGYTNYNEKFK | SEQ ID NO: 27 |
| | CDRH3-Kabat | SDYRFDEGFAY | SEQ ID NO: 28 |
| Light chain | CDRL1-Kabat | SASSSVSSSYLH | SEQ ID NO: 29 |
| | CDRL2-Kabat | RTSNLAS | SEQ ID NO: 30 |
| | CDRL3-Kabat | QQWSGYPYT | SEQ ID NO: 31 |
| Table 7 shows the CDR sequence of mouse anti-Ang2 antibody 1H10. | | | |

[TABLE 8]

| Domain | Variable region sequence | Sequence number |
|---|---|---|
| Heavy chain | QVQLQQSGAELVRPGTSVKMSCKATGYTFTNYWIGWVKQRPGHGLEWIGDIYPGGGYTNYNEKFKGKATLTADTSSSTACMQLSSLTSEDSAIYYCARSDYRFDEGFAYWGQGTLVTIS | SEQ ID NO: 32 |
| Light chain | ENVLTQSPAIMAASLGQKVTMTCSASSSVSSSYLHWYQQKSGASPKPLIHRTSNLASGVPARFSGSGSGTSYSLTISSVEAEDDATYYCQQWSGYPYTFGGGTKLEIKRA | SEQ ID NO: 33 |
| Table 8 shows the variable region sequence of mouse anti-Ang2 antibody 1H10. | | |

## Example 5: ELISA binding of anti-Ang2 antibody to Human Ang2 or mouse Ang2

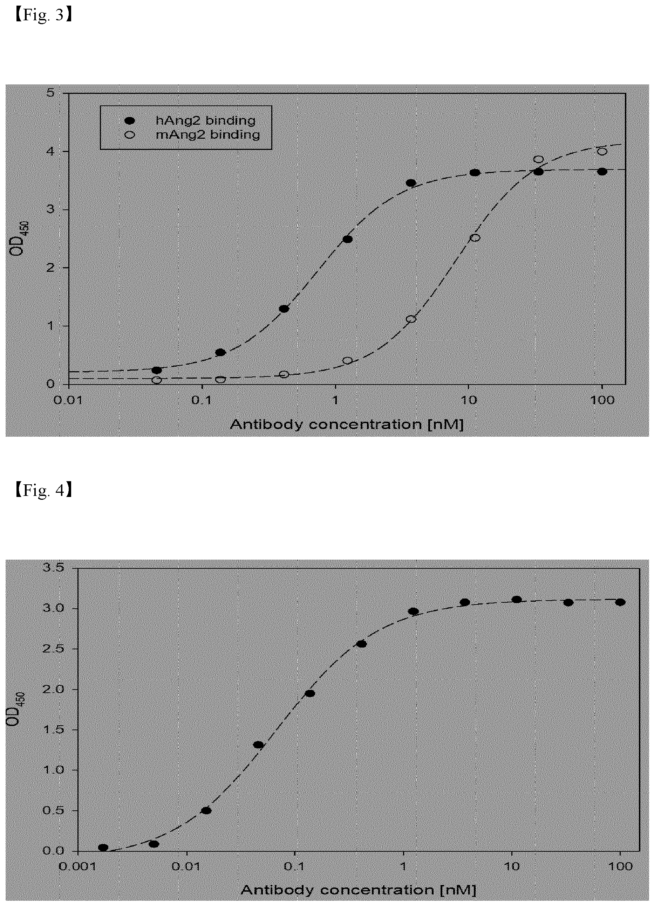

ELISA was performed to confirm the binding ability of anti-Ang2 antibody to human Ang2 or mouse Ang2. A 96-well MaxiSorp™ flat-bottom plate was coated with 1 $\mu$g/mL of human Ang2 (Sino Biological) or mouse Ang2 (Acrobiosystemsl). Then, the plate was washed five times with PBST (Phosphate Buffer Saline containing 0.05% (v/v) Tween-20) and then blocked with PBST containing 1% (v/v) skim milk at room temperature for 2 hours. Afterwards, the plate was washed five times with PBST containing 0.05% Tween-20, and anti-Ang2 serially 3-fold diluted was added at 10 different concentrations starting from 300 nM and allowed to bind at room temperature for 2 hours. After washing the plate 5 times with 0.05% Tween-20 in PBST, Goat anti-Mouse IgG/HRP (Solarbio) diluted 1:3000 in 1% skim milk was reacted for 1 hour and washed 6 times with PBST. Finally, 100 $\mu$L (microliter) of TMB substrate (Kementec) was added to the plate to induce a color reaction for 10 minutes. Afterwards, 100 $\mu$L of Stop solution (2 M H2SO4) was added to stop the reaction, and the OD450 value was measured on a plate reader (BioTek). The obtained results are shown in Figure 3.

## Example 6: ELISA for confirmation of antibody-Ang2-Tie2 complex formation of anti-Ang2 antibody

ELISA was performed to determine whether a complex was formed between anti-Ang2 antibody and Ang2 Tie2 receptor. A 96-well MaxiSorp™ flat-bottom plate was coated with 2 $\mu$g/ml of Tie2 ECD (Sino Biological). Then, the plate was washed five times with PBST (Phosphate Buffer Saline containing 0.05% (v/v) Tween-20) and then blocked with PBST containing 1% (v/v) skim milk at room temperature for 2 hours. Afterwards, the plate was washed five times with PBST containing 0.05% Tween-20, and the previously prepared anti-Ang2 antibody and Ang2 complex were added and allowed to bind to Tie2 for 2 hours at room temperature. Anti-Ang2 antibody and Ang2 complex were prepared by adding anti-Ang2 antibody to 1% skim milk at a concentration of 600 nM, serially diluting 3-fold using 1% skim milk to prepare 10 different concentrations, and Ang2 was added to 1% skim milk. It was prepared by mixing 1: 1 with the 1 ug/mL diluted sample. After washing the plate 5 times with 0.05% Tween-20 in PBST, Goat anti-Mouse IgG/HRP (Solarbio) diluted 1:3000 in 1% skim milk was reacted for 1 hour and washed 6 times with PBST. Finally, 100 $\mu$L (microliter) of TMB substrate (Kementec) was added to the plate to induce a color reaction for 10 minutes. Afterwards, 50 $\mu$L of Stop solution (2 M H2SO4) was added to stop the reaction, and the OD450 value was measured on a plate reader (BioTek). The obtained results are shown in Figure 4.

## Example 7: Competition ELISA of anti-Ang2 antibody for Ang2-Tie2 binding

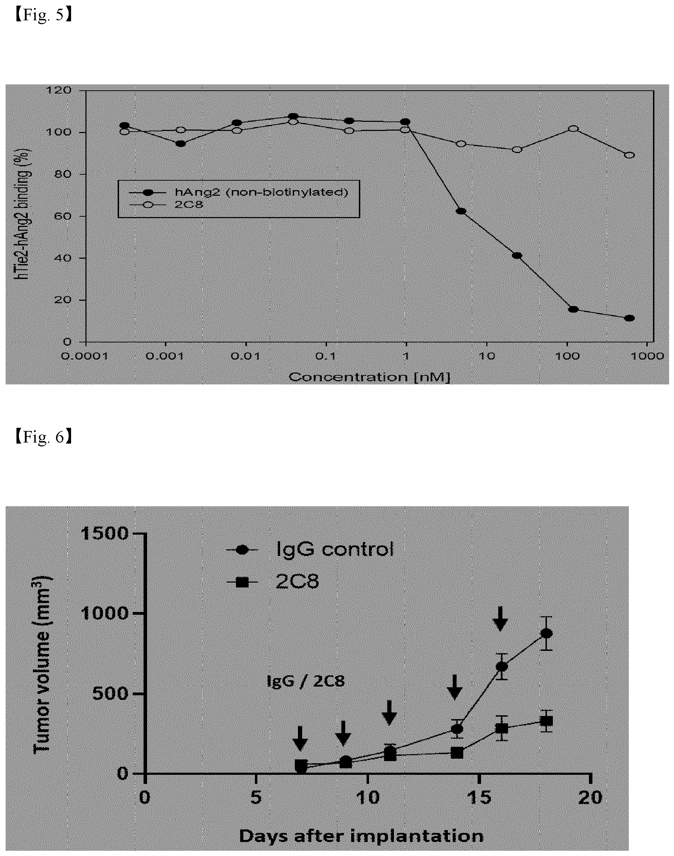

Ang2-Tie2 binding competition ELISA was performed using an anti-Ang2 antibody. More specifically, 96-well MaxiSorp™ flat-bottom plates were coated with 4 $\mu$g/ml Tie2 ECD (Sino Biological). Then, the plate was washed five times with PBST (Phosphate Buffer Saline containing 0.05% (v/v) Tween-20) and blocked with PBST containing 1% (v/v) BSA for 2 hours at room temperature. Afterwards, the plate was washed five times with PBST containing 0.05% Tween-20, and then biotin-labeled human Ang2 and anti-Ang2 antibody complex or biotin-labeled human Ang2 and unlabeled human Ang2 mixture were added and was allowed to bind to Tie2 at room temperature for 2 hours. At this time, for biotin-labeled human Ang2, mix 1 $\mu$l of modifier reagent with 10 $\mu$l of human Ang2 (>1mg/ml), remove the cap of the vial containing biotin conjugation mix, add Ang2-modifier solution, mix, and after blocking light at room temperature, it was prepared by reacting for more than 15 minutes and then adding 1 $\mu$l Quencher reagent and reacting for 5 minutes. Anti-Anti-Ang2 antibody and biotin-labeled Ang2 complex were prepared by adding anti-Ang2 antibody at a concentration of 1.2 $\mu$M to 1% BSA and serially diluting 5-fold using 1% BSA to prepare 10 different concentrations and then mixed biotin-labeled human Ang2 1:1 with a sample diluted to 1 $\mu$g/mL in 1% BSA. After washing the plate 5 times with 0.05% Tween-20 in PBST, streptavidin HRP (Abcam) diluted 1:10000 in 1% BSA was bound for 1 hour and washed 6 times with PBST. Finally, 100 $\mu$L (microliter) of TMB substrate (Kementec) was added to the plate to induce a color reaction for 10 minutes. Afterwards, 50 $\mu$L of Stop solution (2 M $H_2SO_4$) was added to stop the reaction, and the OD450 value was measured on a plate reader (BioTek). The obtained results are shown in Figure 5.

**Example 8: Tumor growth inhibition effect of anti-Ang2 antibody in mouse cancer model**

To confirm the tumor growth inhibition effect of anti-Ang2 antibody, a mouse tumor model was used. Tumor volume (V) was calculated using the following equation:

$$V = \text{Tumor volume (mm}^3) = (\text{length x width}^2)/2$$

A. LLC lung cancer cell tumor model

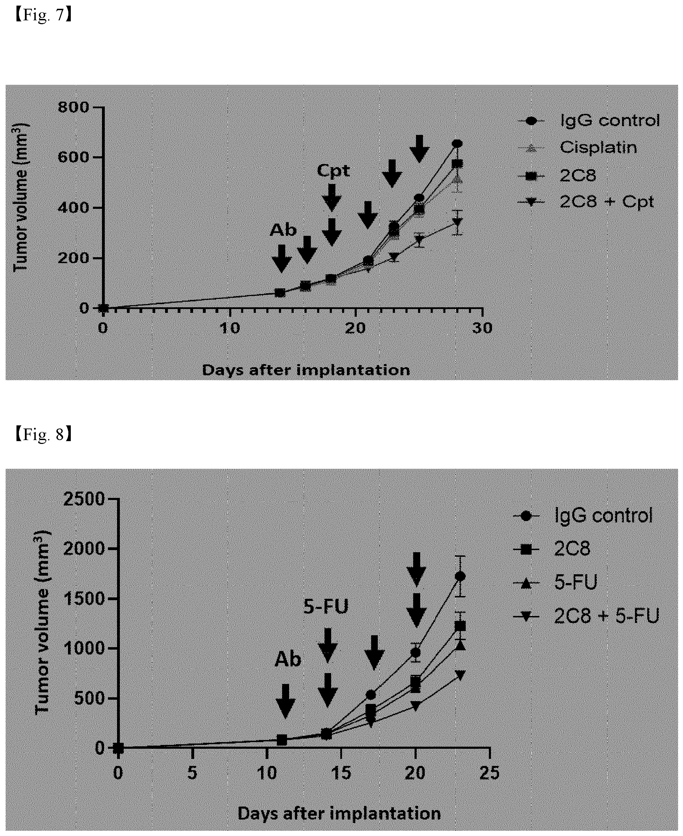

To confirm the tumor growth inhibition effect of the anti-Ang2 antibody, a syngeneic lung cancer mouse model using the LLC (Lewis lung carcinoma, ATCC) cell line, a mouse lung cancer cell line, was used. The LLC cell line was cultured in DMEM (Welgene) supplemented with 10% FBS (Gibco). After LLC cells ($1 \times 10^6$ in 100 $\mu$L of PBS + 100 $\mu$L matrigel) were subcutaneously inoculated into C57BL/6 mice(from Central Laboratory Animal Company, South Korea) of the same genotype, 5 mg of anti-Ang2 antibody (2C8) was intraperitoneally administered on days 7, 9, 11, 14, and 16 from the date of cancer cell inoculation. And the volume of the tumor was measured.
As shown in Figure 6, it was confirmed that anti-Ang2 antibody 2C8 inhibited tumor growth.
To confirm the effect of combined administration of anti-Ang2 antibody with other existing anticancer drugs, a combined administration test of Ang2 antibody 2C8 and cisplatin was performed in LLC lung cancer model. The LLC cell line was cultured in DMEM (Welgene) supplemented with 10% FBS (Gibco). After LLC cells ($1 \times 10^6$ in 100 $\mu$L of PBS) were subcutaneously inoculated into C57BL/6 mice(from ORIENT Company, South Korea) of the same genotype, Ang2 antibody was administered intraperitoneally at a dose of 5 mg/kg three times a week for 2 weeks, when the tumor volume reached 50-100 mm$^3$. Cisplatin (Sigma) was administered once at a dose of 5 mg/kg. The experiment was performed in Ig (control group), 2C8 group, cisplatin group, and 2C8+cisplatin group.
As shown in Figure 7, when anti-Ang2 antibody 2C8 and cisplatin were co-administered, tumor growth was inhibited by about 48% compared to the control, showing significantly more significant efficacy than anti-Ang2 antibody or 5FU alone. These results show that anti-Ang2 antibodies, when administered in combination with other anticancer drugs, can inhibit cancer growth much more powerfully than treatment with existing anticancer drugs alone.

B. MC38 colon cancer cell growth model

To confirm the tumor growth inhibition effect of anti-Ang2 antibody in a colon cancer model and the effect of combined administration with 5FU (5-fluorouracil), a syngeneic mouse model of colon cancer using the MC38 cell line, a mouse colon cancer cell line, was used. MC38 cell line was cultured in DMEM (Welgene) supplemented with 10% FBS (Welgene). After MC38 cells ($1 \times 10^6$ in 100 $\mu$L of PBS) were subcutaneously inoculated into C57BL/6 mice(from ORIENT Company, South Korea) of the same genotype, Ang2 antibody (2C8) was administered intraperitoneally at a dose of 5 mg/kg a total of 5 times at 3-day intervals, when the tumor volume reached 70-100 mm$^3$. 5FU was administered twice at a dose of 20 mg/kg. The experiment was performed in Ig (control group), 2C8 group, 5FU (Sigma) group, and 2C8+5FU group.
As shown in Figure 8, not only anti-Ang2 antibodies 2C8 and 5FU showed a similar level of tumor growth inhibition effect alone, but when anti-Ang2 antibodies 2C8 and 5FU were administered together, tumor growth was inhibited by about 58% compared to the control, thereby inhibiting anti-Ang2 antibodies by about 58%. It showed significantly more significant efficacy compared to Ang2 antibody or 5FU alone. These results show that anti-Ang2 antibodies, when administered in combination with other anticancer drugs, can inhibit cancer growth much more powerfully than treatment with existing anticancer drugs alone.

**Claims**

1. An antibody or antigen-binding fragment thereof that specifically binds to angiopoietin 2 (Ang2) and induces Tie2 activation, comprising:

   (a) a heavy chain complementarity determining region (CDR) comprising CDRH1 of the amino acid sequence of SEQ ID NO: 2, SEQ ID NO: 10, SEQ ID NO: 18, or SEQ ID NO: 26,

   CDRH2 of the amino acid sequence of SEQ ID NO: 3, SEQ ID NO: 11, SEQ ID NO: 19, or SEQ ID NO: 27, and CDRH3 of the amino acid sequence of SEQ ID NO: 4, SEQ ID NO: 12, SEQ ID NO: 20, or SEQ ID NO: 28; and

   (b) a light chain CDR comprising CDRL1 of the amino acid sequence of SEQ ID NO: 5, SEQ ID NO: 13, SEQ ID NO: 21, or SEQ ID NO: 29,

   CDRL2 of the amino acid sequence of SEQ ID NO: 6, SEQ ID NO: 14, SEQ ID NO: 22, or SEQ ID NO: 30, and CDRL3 of the amino acid sequence of SEQ ID NO: 7, SEQ ID NO: 15, SEQ ID NO: 23, or SEQ ID NO: 31.

2. The antibody or antigen-binding fragment thereof of claim 1, wherein the antibody or antigen-binding fragment thereof comprises:

   (a) a heavy chain complementarity determining region (CDR) comprising CDRH1 of the amino acid sequence of SEQ ID NO: 2, CDRH2 of the amino acid sequence of SEQ ID NO: 3, and CDRH3 of SEQ ID NO: 4; and
   (b) a light chain CDR comprising CDRL1 of the amino acid sequence of SEQ ID NO: 5, CDRL2 of the amino acid sequence of SEQ ID NO: 6, and CDRL3 of the amino acid sequence of SEQ ID NO: 7.

3. The antibody or antigen-binding fragment thereof of claim 1, wherein the antibody or antigen-binding fragment thereof comprises:

   (a) a heavy chain complementarity determining region (CDR) comprising CDRH1 of the amino acid sequence of SEQ ID NO: 10, CDRH2 of the amino acid sequence of SEQ ID NO: 11, and CDRH3 of SEQ ID NO: 12; and
   (b) a light chain CDR comprising CDRL1 of the amino acid sequence of SEQ ID NO: 13, CDRL2 of the amino acid sequence of SEQ ID NO: 14, and CDRL3 of the amino acid sequence of SEQ ID NO: 15.

4. The antibody or antigen-binding fragment thereof of claim 1, wherein the antibody or antigen-binding fragment thereof comprises:

   (a) a heavy chain complementarity determining region (CDR) comprising CDRH1 of the amino acid sequence of SEQ ID NO: 18, CDRH2 of the amino acid sequence of SEQ ID NO: 19, and CDRH3 of SEQ ID NO: 20; and
   (b) a light chain CDR comprising CDRL1 of the amino acid sequence of SEQ ID NO: 21, CDRL2 of the amino acid sequence of SEQ ID NO: 22, and CDRL3 of the amino acid sequence of SEQ ID NO: 23

5. The antibody or antigen-binding fragment thereof of claim 1, wherein the antibody or antigen-binding fragment thereof comprises:

   (a) a heavy chain complementarity determining region (CDR) comprising CDRH1 of the amino acid sequence of SEQ ID NO: 26, CDRH2 of the amino acid sequence of SEQ ID NO: 27, and CDRH3 of SEQ ID NO: 28; and
   (b) a light chain CDR comprising CDRL1 of the amino acid sequence of SEQ ID NO: 29, CDRL2 of the amino acid sequence of SEQ ID NO: 30, and CDRL3 of the amino acid sequence of SEQ ID NO: 31

6. The antibody or antigen-binding fragment thereof of claim 1, wherein the antibody or antigen-binding fragment thereof comprises:
   a heavy chain variable region comprising an amino acid sequence selected from the group consisting of SEQ ID NO: 8, SEQ ID NO: 16, SEQ ID NO: 24, and SEQ ID NO: 32, and a light chain variable region comprising an amino acid sequence selected from the group consisting of SEQ ID NO: 9. , SEQ ID NO: 17, SEQ ID NO: 25, and SEQ ID NO: 33

7. The antibody or antigen-binding fragment thereof of claim 1, wherein the antibody or fragment thereof specifically binds to angiopoietin-2 (Ang2) and binds to the Tie2 receptor together with Ang2.

8. An isolated nucleic acid encoding the anti-Ang2 antibody or antigen-binding fragment thereof of any one of claims 1 to 7.

9. The antibody or antigen-binding fragment thereof of any one of claims 1 to 7, wherein the antigen-binding fragment is selected from the group consisting of scFv, (scFv)$_2$, scFv-Fc, Fab, Fab', and F(ab')$_2$.

10. The antibody or antigen-binding fragment thereof of any one of claims 1 to 7, wherein the antibody or antigen-binding fragment thereof is a murine antibody, chimeric antibody, or humanized antibody.

11. A pharmaceutical composition for the prevention or treatment of diseases associated with Ang2 overexpression, neovascularization, or increased vascular permeability, comprising the anti-Ang2 antibody or antigen-binding fragment thereof of any one of claims 1 to 7 as an active ingredient.

12. The pharmaceutical composition according to claim 11, wherein the disease associated with Ang2 overexpression, neovascularization, or increased vascular permeability is cancer, cancer metastasis, inflammatory disease, infection, cardiovascular disease, kidney disease, hereditary hemorrhagic telangiectasia, asthma, or edema.

13. A pharmaceutical composition for the prevention or treatment of diseases associated with reduced normal blood vessel formation, comprising the anti-Ang2 antibody or antigen-binding fragment thereof of any one of claims 1 to 7 as an active ingredient.

14. The pharmaceutical composition of claim 13, wherein the disease associated with decreased normal vascularization is myocardial infarction, angina pectoris, cerebral infarction, stroke, Buerger's disease, avascular necrosis, foot ulcer, or erectile dysfunction.

15. A pharmaceutical composition for the prevention or treatment of Alzheimer's disease; inflammatory autoimmune disease such as rheumatoid arthritis, multiple sclerosis, or psoriasis; age-related macular degeneration (AMD), eye diseases such as diabetic macular edema (DME), diabetic retinopathy (DR), or glaucoma; or coronavirus infectious diseases such as COVID-19, comprising the anti-Ang2 antibody or antigen-binding fragment thereof of any one of claims 1 to 7 as an active ingredient.

16. A composition for combination therapy for use in cancer treatment comprising; administering the anti-Ang2 antibody or antigen-binding fragment thereof of any one of claims 1 to 7 simultaneously, separately, or sequentially with irradiation, a chemotherapy agent, a cytotoxic agent, and/or an immune-toxic agent.

【Fig. 1】

【Fig. 2】

【Fig. 3】

【Fig. 4】

【Fig. 5】

【Fig. 6】

【Fig. 7】

【Fig. 8】

## INTERNATIONAL SEARCH REPORT

| International application No. |
|---|
| **PCT/KR2022/003351** |

| **A.** | **CLASSIFICATION OF SUBJECT MATTER** |
|---|---|

**C07K 16/22**(2006.01)i; **A61P 35/00**(2006.01)i; **A61P 25/28**(2006.01)i; **A61P 27/02**(2006.01)i; **C07K 16/28**(2006.01)i; **A61K 39/00**(2006.01)i

According to International Patent Classification (IPC) or to both national classification and IPC

| **B.** | **FIELDS SEARCHED** |
|---|---|

Minimum documentation searched (classification system followed by classification symbols)

C07K 16/22(2006.01); A61K 38/18(2006.01); A61K 39/00(2006.01); A61K 39/395(2006.01); A61K 45/06(2006.01); C07K 16/28(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 안지오포이에틴-2(Angiopoietin-2), Tie2, 항체(antibody), 암(cancer), 치료 (treatment)

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| X | KR 10-2015-0136031 A (SAMSUNG ELECTRONICS CO., LTD.) 04 December 2015 (2015-12-04)<br>See paragraphs [0006], [0126], [0143], [0165] and [0168]-[0169]; and claims 1, 4-6 and 8-9. | 1,6-16 |
| A | | 2-5 |
| X | US 2015-0079112 A1 (SAMSUNG ELECTRONICS CO., LTD.) 19 March 2015 (2015-03-19)<br>See paragraphs [0053]-[0054], [0083], [0089], [0092], [0108] and [0116]; and claims 1-2, 5-7 and 10. | 1,6-16 |
| X | KR 10-2019-0100060 A (INSTITUTE FOR BASIC SCIENCE et al.) 28 August 2019 (2019-08-28)<br>See abstract; and claims 1, 6-7 and 16-23. | 1,6-16 |
| A | WO 2013-028442 A1 (REGENERON PHARMACEUTICALS, INC.) 28 February 2013 (2013-02-28)<br>See entire document. | 1-16 |

☑ Further documents are listed in the continuation of Box C.  ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **10 June 2022** | **10 June 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

# EP 4 328 242 A1

## INTERNATIONAL SEARCH REPORT

| | International application No. |
|---|---|
| | **PCT/KR2022/003351** |

**C.    DOCUMENTS CONSIDERED TO BE RELEVANT**

| Category* | Citation of document, with indication, where appropriate, of the relevant passages | Relevant to claim No. |
|---|---|---|
| A | EP 2679599 B1 (AMGEN INC.) 03 January 2018 (2018-01-03) See entire document. | 1-16 |

Form PCT/ISA/210 (second sheet) (July 2019)

22

## INTERNATIONAL SEARCH REPORT

| International application No. |
| --- |
| **PCT/KR2022/003351** |

**Box No. I          Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑ forming part of the international application as filed:

        ☑ in the form of an Annex C/ST.25 text file.

        ☐ on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (July 2019)

**INTERNATIONAL SEARCH REPORT**
**Information on patent family members**

International application No.

**PCT/KR2022/003351**

| Patent document cited in search report | | | Publication date (day/month/year) | Patent family member(s) | | | Publication date (day/month/year) |
|---|---|---|---|---|---|---|---|
| KR | 10-2015-0136031 | A | 04 December 2015 | KR | 10-2349370 | B1 | 10 January 2022 |
| | | | | US | 10934350 | B2 | 02 March 2021 |
| | | | | US | 2015-0337033 | A1 | 26 November 2015 |
| | | | | US | 2018-0273613 | A1 | 27 September 2018 |
| | | | | US | 9994632 | B2 | 12 June 2018 |
| US | 2015-0079112 | A1 | 19 March 2015 | EP | 2848631 | A1 | 18 March 2015 |
| | | | | EP | 2848631 | B1 | 05 September 2018 |
| | | | | EP | 3272772 | A1 | 24 January 2018 |
| | | | | EP | 3272772 | B1 | 04 September 2019 |
| | | | | KR | 10-2015-0032075 | A | 25 March 2015 |
| | | | | KR | 10-2196450 | B1 | 30 December 2020 |
| | | | | US | 9505841 | B2 | 29 November 2016 |
| KR | 10-2019-0100060 | A | 28 August 2019 | CN | 111741975 | A | 02 October 2020 |
| | | | | EP | 3755714 | A1 | 30 December 2020 |
| | | | | JP | 2021-514670 | A | 17 June 2021 |
| | | | | US | 2021-0079083 | A1 | 18 March 2021 |
| | | | | WO | 2019-164219 | A1 | 29 August 2019 |
| WO | 2013-028442 | A1 | 28 February 2013 | CN | 103874709 | A | 18 June 2014 |
| | | | | CN | 106963946 | A | 21 July 2017 |
| | | | | EP | 2744827 | A1 | 25 June 2014 |
| | | | | EP | 2744827 | B1 | 03 October 2018 |
| | | | | EP | 3415533 | A1 | 19 December 2018 |
| | | | | JP | 2014-525934 | A | 02 October 2014 |
| | | | | JP | 2017-186347 | A | 12 October 2017 |
| | | | | JP | 6333174 | B2 | 30 May 2018 |
| | | | | JP | 6654165 | B2 | 26 February 2020 |
| | | | | KR | 10-2014-0054303 | A | 08 May 2014 |
| | | | | US | 10023641 | B2 | 17 July 2018 |
| | | | | US | 10316105 | B2 | 11 June 2019 |
| | | | | US | 10442864 | B2 | 15 October 2019 |
| | | | | US | 10752702 | B2 | 25 August 2020 |
| | | | | US | 2013-0209492 | A1 | 15 August 2013 |
| | | | | US | 2015-0197578 | A1 | 16 July 2015 |
| | | | | US | 2017-0088622 | A1 | 30 March 2017 |
| | | | | US | 2017-0174789 | A1 | 22 June 2017 |
| | | | | US | 2018-0291105 | A1 | 11 October 2018 |
| | | | | US | 2019-0276559 | A1 | 12 September 2019 |
| | | | | US | 9017670 | B2 | 28 April 2015 |
| | | | | US | 9546218 | B2 | 17 January 2017 |
| EP | 2679599 | B1 | 03 January 2018 | CN | 102046657 | A | 04 May 2011 |
| | | | | EP | 2252632 | A1 | 24 November 2010 |
| | | | | EP | 2252632 | B1 | 15 January 2014 |
| | | | | EP | 2679599 | A1 | 01 January 2014 |
| | | | | JP | 2011-525104 | A | 15 September 2011 |
| | | | | JP | 5739163 | B2 | 24 June 2015 |
| | | | | KR | 10-1600639 | B1 | 07 March 2016 |
| | | | | KR | 10-2010-0118143 | A | 04 November 2010 |
| | | | | US | 10336820 | B2 | 02 July 2019 |
| | | | | US | 2009-0226447 | A1 | 10 September 2009 |
| | | | | US | 2012-0034237 | A1 | 09 February 2012 |

Form PCT/ISA/210 (patent family annex) (July 2019)

**INTERNATIONAL SEARCH REPORT**
Information on patent family members

International application No.

**PCT/KR2022/003351**

| Patent document cited in search report | Publication date (day/month/year) | Patent family member(s) | | Publication date (day/month/year) |
|---|---|---|---|---|
| | | US | 2012-0258122 A1 | 11 October 2012 |
| | | US | 2013-0336992 A1 | 19 December 2013 |
| | | US | 2017-0081398 A1 | 23 March 2017 |
| | | US | 8030025 B2 | 04 October 2011 |
| | | US | 8221749 B2 | 17 July 2012 |
| | | WO | 2009-105269 A1 | 27 August 2009 |

Form PCT/ISA/210 (patent family annex) (July 2019)

REFERENCES CITED IN THE DESCRIPTION

**Patent documents cited in the description**

- KR 1020150136031

- US 10934350 B

**Non-patent literature cited in the description**

- **KABAT et al.** *Ann. NY Acad, Sci,* 1971, vol. 190, 382-391
- **KABAT, E. A. et al.** Sequences of proteins of immunological interest. Department of Health and Human Services, National Institutes of Health, 1991
- **SCHWABER, J ; COHEN, E. P.** Human x Mouse Somatic Cell Hybrid Clones Secreting Immunoglobulins of Both Parental Types. *Nature,* 1973, vol. 244, 444-447

- **ALMAGRO, J. C. ; FRANSSON, J.** Humanization of antibodies. *Frontiers in Bioscience,* 2008, vol. 13, 1619-1633
- **KOHLER ; MILSTEIN.** *Eur. J Immunol.,* 1976, vol. 6, 511
- **MEYER L et al.** A simplified workflow for monoclonal antibody sequencing. *PLoS ONE,* 2019